Europäisches Patentamt

(19) European Patent Office

Office européen des brevets

(11) Veröffentlichungsnummer: **0 299 090**
**A1**

(12) **EUROPÄISCHE PATENTANMELDUNG**

Veröffentlicht nach Art. 158 Abs. 3 EPÜ

(21) Anmeldenummer: **88901396.7**

(22) Anmeldetag: **28.12.87**

Daten der zugrundeliegenden internationalen Anmeldung:

(86) Internationale Anmeldenummer:
**PCT/SU87/00157**

(87) Internationale Veröffentlichungsnummer:
**WO88/05292 (28.07.88 88/17)**

(30) Priorität: **27.01.87 SU 4185874**

(43) Veröffentlichungstag der Anmeldung:
**18.01.89 Patentblatt 89/3**

(84) Benannte Vertragsstaaten:
**BE CH DE FR GB IT LI NL SE**

(51) Int. Cl.³: **A 61 F 5/41**

(71) Anmelder: **VSESOJUZNY NAUCHNO-ISSLEDOVATELSKY I ISPYTATELNY INSTITUT MEDITSINSKOI TEKHNIKI**
**ul. Kasatkina, 3**
**Moscow, 129301(SU)**

(72) Erfinder: **ZUSMANOVSKY, Zinovy Abramovich**
**5 Sovetskaya ul., 34-42**
**Leningrad, 193144(SU)**

(72) Erfinder: **TSIRJULNIKOV, Moisei Vulfovich**
**Vasilievsky ostrov, 15-14-8**
**Leningrad, 199178(SU)**

(74) Vertreter: **Patentanwälte Beetz sen. - Beetz jun. Timpe - Siegfried - Schmitt-Fumian- Mayr**
**Steinsdorfstrasse 10**
**D-8000 München 22(DE)**

(54) **ANORDNUNG ZUR HEILUNG DER MÄNNLICHEN IMPOTENZ.**

(57) Die Vorrichtung zum Heilen der Impotenz bei Männern enthält parallel verlaufende, miteinander bewegbar verbundene und um ihre Achsen drehbare Stäbe (1), deren jeder mit einem der Enden von bogenförmigen Elementen (3,5,7,8) verbunden ist, welche einen aus gebogenen Platten hergestellten Feststeller (4) für die Eichel des männlichen Gliedes, einen Steuereungsfeststeller (6) und einen Basisfeststeller (10) bilden, wobei die freien Enden der Elemente (7,8) des letzteren durch ein elastisches Element (9) miteinander verbunden sind, welches eine Einrichtung zum umkehrbaren Regulieren seines Spannungsgrades und eines Öse (12), aufweist, welche Öse aus dem gleichen Werkstoff wie das elastische Element (9) hergestellt ist.

Croydon Printing Company Ltd.

EP 0 299 090 A1

# VORRICHTUNG ZUM HEILEN DER IMPOTENZ BEI MÄNNERN

## Gebiet der Technik

Die vorliegende Erfindung bezieht sich auf das Gebiet der Medizin und betrifft insbesondere Vorrichtungen zum Heilen der Impotenz bei Männern.

## Zugrundeliegender Stand der Technik

Breit bekannt sind Vorrichtungen zum Heilen der Impotenz bei Männern, welche Bedingungen schaffen, unter denen die Erektion des männlichen Gliedes sichergestellt wird, und einen Haltestab enthalten, der an einen Stützring angelenkt ist und mit weichen Bügeln abschließt.

Bekannt ist eine Vorrichtung zum Heilen der Impotenz (SU,A,178044), die zwei aneinander anstoßende und mit einer gemeinsamen elastischen Hülle überzogene Stäbe enthält. Von distaler Seite her enden die Stäbe mit Bügeln, welche die Form der Kranzfurche wiederholen und die Eichel umgreifen, während sie von proximaler Seite her mit Segmenten enden, die bei deren Drehen gemeinsam mit einem regulierbaren Teil einen Stützring bilden, der sich gegen den Schamhügel stützt.

Ein Nachteil dieser Vorrichtung besteht darin, daß keine Möglichkeit besteht, den Kompressionsgrad der Eichel des männlichen Gliedes in Abhängigkeit vom dem Grad der aufkommenden echten Erektion während des Geschlechtsaktes zu regulieren, sowie daß die individuelle Anpassung umständlich ist.

Diese Nachteile sind in gewissem Maße in einer Vorrichtung zum Heilen der Impotenz (SU, A, 589978) behoben, die parallele Stäbe enthält, die beweglich miteinander verbunden und um ihre Achsen drehbahr sind, wobei jeder der Stäbe mit einem der Enden von bogenförmigen Elementen verbunden ist, welche einen Eichelfeststeller, einen Steuerungsfeststeller und einen Basisfeststeller bilden, wobei die freien Enden der bogenförmigen Elemente des letzteren miteinander durch ein elastisches Element mit einer Öse verbunden sind, die mit einem am freien Ende eines der bogenförmigen Elemente des Basisfeststellers befestigten Haken zusammenwirkt.

Die bekannte Vorrichtung gewährleistet eine dosierte Kompresion der Eichel des männlichen Gliedes durch Lappen des Feststellers in Abhängigkeit von dem Schwellungsgrad des Gliedes, wodurch ein schmerzhaftes Empfinden wegen Zusammendrücken der Eichel des erigierten Gliedes durch den Feststeller beim Aufkommen der Erektion ausgeschlossen wird und somit ein Effekt der Abwesenheit von Hilfsmitteln auftritt.

Diese bekannte Vorrichtung gewährleistet jedoch nicht eine maximal sichere Fixierung der Eichel des männlichen Gliedes, weil die Bewehrung des vorderen Feststellers einen runden Querschnitt aufweist, die Anliegefläche des betreffenden Feststellers am Glied minimal ist, so daß sich die elastische Hülle durchdrehen kann, was zum Schlüpfen der Eichel des männlichen Gliedes aus dem zusammengeschlossenen Feststeller führt. Zum anderen ist bei der bekannten Vorrichtung zum Heilen der Impotenz zu bemängeln, daß eine maximale Genauigkeit der individuellen Anpassung nicht gewährleistet ist. Das Anpassen der Länge der Kupplung im Sinne der Kürzung sieht das Abschneiden eines überschüssigen Stückes vor, wodurch die Rückkehr in die frühere Lage bei der Fehlanpassung ausgeschlossen ist.

Die primäre Anpassung der Vorrichtung, welche an einem schlaffen Glied vorgenommen wird, kann sich beim Aufkommen der Erektion während des Geschlechtsaktes als ungenügend genau erweisen. Bei der bekannten Vorrichtung wird die Regulierung durch Kürzen der Länge des elastischen Elementes vorgenommen, was eine richtige Anpassung nicht gewährleistet und bei dem Patienten eine Dyskomfortempfindung während des Geschlechtsaktes aufkommen läßt und somit die Wirksamkeit der Heilung herabsetzt. Der runde Querschnitt der bogenförmigen Elemente des Eichelfeststellers kann ebenfalls einen Dyskomport bei dem Aufkommen der Erektion hervorrufen, was mit einem großen spezifischen Druck auf die Gewebe des männlichen Gliedes im Bereich der Kranzfurche zusammenhängt, und gewährleistet ebenso nicht eine sichere Fixierung der Eichel, und zwar deshalb, weil die Anliegefläche der bogenförmigen Elemente des Feststellers an der Haut des männlichen Gliedes gering ist, was äußerst ungünstig für die Behandlungseffektivität ist und bei dem Patienten ein Mißtrauen gegen die gewählte Behandlungsmethode hervorrufen kann.

Darüber hinaus verursacht die am Ende des elastischen Elementes befestigte und aus einem nichtelastischen Werkstoff hergestellte Öse einen zusätzlichen Dyskomfort für die Partner während des Geschlechtsaktes.

Offenbarung der Erfindung

Der Erfindung liegt die Aufgabe zugrunde, eine Vorrichtung zum Heilen der Impotenz bei Männern zu schaffen, die eine umkehrbare Regulierung des Spannungsgrades des elastischen Elementes des Basisfeststellers und eine Herabsetzung des spezifischen Druckes auf die Gewebe des männlichen Gliedes im Bereich der Kranzfurche sowie eine erhöhte Sicherheit der Fixierung der Eichel des nicht erigierten Gliedes gewährleistet.

Die gestellte Aufgabe wird mit Hilfe einer Vorrichtung gelöst, die parallel verlaufende, miteinander bewegbar verbundene und um ihre Achsen drehbare Stäbe enthält, deren jeder mit einem der Enden von bogenförmigen Elementen verbunden ist, welche einen Feststeller für die Eichel des männlichen Gliedes, einen Steuerungsfeststeller und einen Basisfeststeller bilden, wobei die freien Enden der bogenförmigen Elemente des letzteren miteinander durch ein elastisches Element mit einer Öse verbunden sind, welche Öse mit einem am freien Ende eines der bogenförmigen Elemente des Basisfeststellers befestigten Haken zusammenwirkt, und bei welcher Vorrichtung erfindungsgemäß das elastische Element mit einer Einrichtung zum umkehrbaren Regulieren seines Spannungsgrades versehen ist, wobei die Öse aus dem gleichen Werkstoff wie das elastische Element hergestellt ist und die bogenförmigen Elemente des Eichelfeststellers in Form von gebogenen Platten ausgebildet sind.

Eine solche Ausführung der Vorrichtung erhöht die Wirksamkeit der Behandlung, indem sie die Spannung des elastischen Elementes des Basisfeststellers genau regulieren läßt, den spezifischen Druck auf die Gewebe der Kranzfurche des männlichen Gliedes verringert und gleichzeitig die Sicherheit der Fixierung der Eichel erhöht.

Die Einrichtung zum umkehrbaren Regulieren wird zweckmäßigerweise in Form von kugelförmigen Verdickungen am elastischen Element ausgebildet, welche mit einem Verschluß zusammenwirken, der am freien Ende des anderen bogenförmigen Elementes des Basisfeststellers angeordnet ist und zwei durch einen Schlitz miteinander verbundene Öffnungen aufweist, wobei der Durchmesser einer der Öffnungen größer und der Durchmesser der anderen kleiner als der Durchmesser der kugelförmigen Verdickungen ist.

Die erfindungsgemäße Ausführung der Einrichtung zum umkehrbaren Regulieren läßt den Spannungsgrad des elastischen Elementes hinreichend genau und schnell regulieren und ist gleichzeitig durch eine einfache konstruktive Lösung gekennzeichnet.

Kurze Beschreibung der Zeichnungen

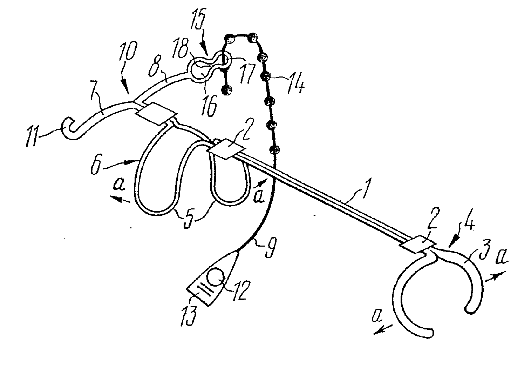

Das Wesen und weitere Vorteile der vorliegenden Erfindung werden in nachfolgender Beschreibung mit Hinweisen auf eine beigelegte Zeichnung näher erläutert. Die Zeichnung zeigt die erfindungsgemäße Vorrichtung zum Heilen der Impotenz bei Männern.

Beste Ausführungsform der Erfindung

Die Vorrichtung zum Heilen der Impotenz bei Männern enthält zwei parallele Stäbe I, die aus metallischem Draht hergestellt und derart angeordnet sind, daß sie sich um ihre geometrischen Achsen in Kupplungen 2 drehen können. An einem der Enden ist jeder Stab I mit dem Ende eines bogenförmigen Elementes 3 verbunden, welches als eine nach der Form der Kranzfurche des männlichen Gliedes gebogene Platte ausgebildet ist. Die beiden bogenförmigen Elemente 3 bilden gemeinsam einen Feststeller 4 für die Eichel des männlichen Gliedes. Im mittleren Teil sind die Stäbe I mit bogenförmigen Elementen 5 verbunden, die als Schleifen gebogen sind, deren Halbbögen das männliche Glied am Umfang umgreifen, und die einen Steuerungsfeststeller 6 bilden. Am anderen Ende sind die Stabe I mit den Enden von bogenförmigen Elementen 7 und 8 verbunden, die gemeinsam mit einem elastischen Element 9, welches aus Gummi her[g]

- 5 -

stellt ist, einen Basisfeststeller 10 bilden. Das freie Ende des bogenförmigen Elementes 7 ist mit einem Haken II versehen, der mit einer Öffnung in einer an einem der Enden des elastischen Elementes 9 ausgeführten Öse 12 zusammenwirkt. Die Öse 12 ist mit dem elastischen Element 9 einteilig ausgebildet und mit einem Halter 13 versehen. Am anderen Ende des elastischen Elementes 9 sind kugelförmige Verdickungen 14 vorgesehen, die mit einem Verschluß 15 zusammenwirken, der am freien Ende des bogenförmigen Elementes 8 angeordnet ist. Der Verschluß 15 stellt zwei Öffnungen 16 und 17 dar, die miteinander durch einen Schlitz 18 verbunden sind. Der Durchmesser der Öffnung 16 ist größer und der Durchmesser der Öffnung 17 kleiner als der Durchmesser der kugelförmigen Verdickung 14 des elastischen Elementes 9. Die Oberflächen der metallischen Elemente 1, 2, 3, 5, 7, 8 haben eine elastische Umhüllung.

Die Vorrichtung funktioniert wie folgt.

Die Vorrichtung wird auf das schlaffe Glied aufgesetzt. Der Eichelfeststeller 4 umgreift die Kranzfurche des Gliedes, der Steuerungsfeststeller 6 den mittleren Teil des Gliedes, und der Basisfeststeller umgreift mit den bogenförmigen Elementen 7 und 8 und mit dem elastischen Element 9 die Wurzel des Gliedes hinter dem Hodensack am Schoß. Dabei wird die Öse 12 des elastischen Elementes 9 mit Hilfe des Halters 13 auf den Haken 11 des Bogenförmigen Elementes 7 aufgesetzt, und die Regulierung des Spannungsgrades des elastischen Elementes 9 erfolgt durch die Auswahl einer entsprechenden kugelförmigen Verdickung 14, die in den Verschluß 15 eingeführt wird. Die Befestigung der kugelförmigen Verdickung 14 im Verschluß 15 geschieht folgenderweise: das Ende des elastischen Elementes 9 wird bis zur ausgewählten kugelförmigen Verdickung 14 in die größere Öffnung 16 eingeführt und durch den Schlitz 18 in die kleinere Öffnung 17 verschoben, die die kugelförmige Verdickung 14 festhält. Wenn die Länge des elastischen Elementes, die den Spannungsgrad bestimmt, falsch eingestellt ist, so wird das elastische Element 9 in umge-

- 6 -

kehrter Reihenfolge herausgezogen und durch die erneute Auswahl und Festhalten einer anderen kugelförmigen Verdickung 14 verkürzt oder verlängert.

Das männliche Glied kommt also in die Lage, den Geschlechtsakt auszuführen, ohne daß die Partner einen Dyskomfort empfinden.

Falls die Erektion während des Geschlechtsaktes aufkommt, vergrößert sich das Glied im Durchmesser und drückt auf die Bögen des Steuerungsfeststellers 6 (in Pfeilrichtung a - a), was selbsttätig durch das Drehen der Stäbe 1 um ihre Achsen die Bögen 3 des Eichelfeststellers 4 (in Pfeilrichtung a - a) öffnet und somit das Glied bis zu einer im erigierten Zustand natürlichen Länge und Durchmesser ungehemmt vergrößern läßt, ohne das der Mann einen Dyskomfort dabei spürt. Falls die Erektion während des Geschlechtsaktes nachläßt, so bringt das elastische Element 9 des Basisfeststellers 10 dadurch, daß es im aufgesetzten Zustand etwas ausgedehnt ist, den Eichelfeststeller 4, den Steuerungsfeststeller 6 und den Basisfeststeller 10 sinchron in Ausgangsstellung zurück, womit das Glied festgehalten wird und die Möglichkeit der Fortsetzung des Geschlechtsaktes besteht. Das gleiche Arbeitsprinzip der Vorrichtung gewährleistet die Möglichkeit, den Geschlechtsakt fortzusetzen, falls ein vorzeitiger Sammenerguß erfolgt.

Bei Gebrauch der Vorrichtung bleibt das männliche Glied ganz bloß, wodurch seine Rezeptoren für die sexuelle Stimulation zugänglich sind.

Die Vorrichtung arbeitet nach dem Rückkopplungsprinzip, und dies ist deswegen möglich, weil ihre Konstruktion den Steuerungsfeststeller 6 umfaßt, der dem Auffüllungsgrad der Schwellkörper des männlichen Gliedes folgt und dabei selbsttätig Bedingungen für eine Längen- und Durchmesservergrößerung des männlichen Gliedes bei der während des Geschlechtsaktes aufkommenden Erektion schafft, ohne daß der Mann und seine Partnerin einen Dyskomfort empfinden, was seinerseits einen Effekt der Abwesenheit von Hilfsmitteln herbeiführt. Die erfindungsgemäße Vorrichtung hält das männliche Glied beim Nachlassen der Erektion während des Geschlechtsaktes oder nach dem vorzeitigen Samenerguß fest sowie ermöglicht die Ausführung des Geschlechtsaktes sogar bei voller Abwesenheit der wirksamen Funktion bei dem Patienten. Eine für den jeweiligen Patienten richtig angepaßte Vorrichtung gibt die Möglichkeit, eine harmonische intime Nähe praktisch immer zu erzielen.

Gewerbliche Anwendbarkeit

Die zum Patent angemeldete Erfindung kann beim Heilen der Impotenz bei Männern im wesentlichen funktioneller (psychogener) und funktionell-organischer Herkunft verwendet werden.

- 8 -

PATENTANSPRÜCHE

1. Vorrichtung zum Heilen der Impotenz bei Männern, die parallel verlaufende, miteinander bewegbar verbundene und um ihre Achsen drehbare Stäbe (1) enthält, deren jeder mit einem der Enden von bogenförmigen Elementen (3, 5, 7, 8) verbunden ist, welche einen Feststeller (4) für die Eichel des männlichen Gliedes, einen Steuerungsfeststeller (6) und einen Basisfeststeller (10) bilden, wobei die freien Enden der bogenförmigen Elemente (7, 8) des letzteren miteinander durch ein elastisches Element (9) mit einer Öse (12) verbunden sind, welche Öse (12) mit einem am freien Ende eines (7) der bogenförmigen Elemente (7, 8) des Basisfeststellers (10) befestigten Haken (11) zusammenwirkt, dadurch g e k e n n z e i c h n e t , daß das elastische Element (9) mit einer Einrichtung zum umkehrbaren Regulieren seines Spannungsgrades versehen ist, wobei die Öse (12) aus dem gleichen Werkstoff wie das elastische Element (9) hergestellt ist und die bogenförmigen Elemente (3) des Eichelfeststellers (4) in Form von gebogenen Platten ausgebildet sind.

2. Vorrichtung nach Anspruch 1, dadurch g e k e n n z e i c h n e t , daß die Einrichtung zum umkehrbaren Regulieren in Form von kugelförmigen Verdickungen (14) am elastischen Element (9) ausgebildet ist, welche mit einem Verschluß (15) zusammenwirken, der am freien Ende des anderen bogenförmigen Elementes (8) des Basisfeststellers (10) angeordnet ist und zwei durch einen Schlitz (18) miteinander verbundene Öffnungen (16, 17) aufweist, wobei der Durchmesser einer (16) der Öffnungen (16, 17) größer und der Durchmesser der anderen (17) kleiner als der Durchmesser der kugelförmigen Verdickungen (14) ist.

0299090

# INTERNATIONAL SEARCH REPORT

International Application No PCT/SU 87/00157

**I. CLASSIFICATION OF SUBJECT MATTER** (if several classification symbols apply, indicate all) ⁶

According to International Patent Classification (IPC) or to both National Classification and IPC

$IPC^4$: A 61 F 5/41

**II. FIELDS SEARCHED**

| Minimum Documentation Searched ⁷ | |
|---|---|
| Classification System | Classification Symbols |
| $IPC^4$ | A 61 F 5/40-5/42 |

| Documentation Searched other than Minimum Documentation to the Extent that such Documents are Included in the Fields Searched ⁸ |
|---|
| |

**III. DOCUMENTS CONSIDERED TO BE RELEVANT** ⁹

| Category * | Citation of Document, ¹¹ with indication, where appropriate, of the relevant passages ¹² | Relevant to Claim No. ¹³ |
|---|---|---|
| A,P | US, A, 4672954, (Jack S.Panzer), 16 June 1987 (16.06.87), see the abstract, figures 1,2,4 | 1 |
| A | US, A, 4429689, (Procopio U. Yanong), 7 February 1984 (07.02.84), see the abstract, figure 1 | 1 |
| A | GB, A, 2086230, (Francis Ward Allinson), 12 May 1982 (12.05.82), see the abstract, figures 1-16 | 1 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 16 March 1988 (16.03.88) | 28 April 1988 (28.04.88) |
| International Searching Authority | Signature of Authorized Officer |
| ISA/SU | |

Form PCT/ISA/210 (second sheet) (January 1985)